# EUROPEAN PATENT APPLICATION

(11) **EP 3 834 826 A1**
(43) Date of publication of application: **16.06.2021**
(21) Application number: 19847406.6
(22) Date of filing: 09.08.2019
(51) Int. Cl.: A61K 31/436, A61K 31/407, A61K 38/13, A61P 37/06

(54) **THERAPEUTIC AGENT FOR HUMORAL IMMUNITY-RELATED DISEASES IN MATERNO-FETAL RELATIONSHIP**

(30) Priority: 10.08.2018 JP 2018152084
(71) Applicant: Yamaguchi, Koushi, Koganei-shi, Tokyo 184-0004 (JP)
(72) Inventor: Yamaguchi, Koushi, Koganei-shi, Tokyo 184-0004 (JP)
(74) Representative: Schön, Christoph
(86) International application number: PCT/JP2019/031622
(87) International publication number: WO 2020/032252

(57) **Abstract**

A medicine for treating or ameliorating a disease associated with humoral immunity in the materno-fetal relationship, for example, sterility and infertility caused by humoral immunity in the materno-fetal relationship, blood type incompatible pregnancy, or fetal hemochromatosis, the medicine including, as an active ingredient, a compound selected from the group consisting of: (i) a compound represented by Formula (I) (wherein the respective reference numerals are as described in the specification) or a pharmaceutically acceptable salt thereof, (ii) a cyclosporine, and (iii) a rapamycin derivative.

## Description

### Technical Field

The present invention relates to a medicine for treating or ameliorating a disease associated with humoral immunity in the materno-fetal relationship, for example, sterility and infertility caused by humoral immunity in the materno-fetal relationship, blood type incompatible pregnancy, or fetal hemochromatosis, the medicine including a particular immunosuppressant component, and to utilization of the medicine.

### Background Art

Acquired immunity is further classified into "cell-mediated immunity" and "humoral immunity" depending on the type of helper T cells and the way of action. Examples of maternal- and fetal-related diseases or symptoms of the immune system include, for the "cell-mediated immunity", sterility caused by implantation disorder, infecundity caused by placenta development disorder, and pregnancy hypertension; and for the "humoral immunity", sterility and infertility caused by humoral immunity in the materno-fetal relationship, blood type incompatible pregnancy, and fetal hemochromatosis.

With regard to the relationship between immunity and pregnancy, for example, Patent Literature 1 describes a therapeutic drug for sterility and infertility caused by "cell-mediated immunity", the therapeutic drug containing a specific immunosuppressant agent as an active ingredient.

Placenta is configured to avoid mixing of blood between the mother's body and the fetus; however, a small amount of fetal antigens including blood cells enter the maternal circulation via the placenta (feto-maternal transfusion, FMT). FMT occurs most frequently during childbirth [Reference Literatures 1 to 3]; however, in view of fetal antigen recognition, FMT can also occur during all pregnancies, including pregnancies that result in recurrent failure and spontaneous abortion.

Pathogenic antibodies to fetal antigens are produced in the mother in a case where the fetal antigens have antigenicity that exceeds the mother's immunological tolerance capacity and are recognized as foreign antigens. The produced pathogenic antibodies either attack the fetal components that construct the fertilized ovum or the placenta, or migrate to the fetal circulation system via the placenta, similarly to other IgG antibodies, and attack target fetal antigens on the cell membrane or inside and outside the cells in the fetus.

In the case of being frequently exposed to the same fetal antigen, including recurrent failure, habitual miscarriage after the first pregnancy, miscarriage surgery, stillbirth, and childbirth, there is a possibility that more pathogenic antibodies may be produced in proportion to the number of recurrent failures and pregnancies and exist in the mother's body even before pregnancy.

When pathogenic antibodies attack the fertilized ovum at the time of implantation, the attack leads to infecundity, and when pathogenic antibodies attack fetal components that construct the placenta after implantation, the attack leads to miscarriage, so that even if miscarriage is avoided, attacking of fetal components that construct a persistent placenta can lead to infertility.

Even in a case where pathogenic antibodies do not attack fetal components that construct the placenta and do not cause sterility and infertility, the antibodies migrate to the fetal circulation system via the placenta and attack target fetal antigens on the cell membrane or inside and outside the cells of the fetus.

After construction of the placenta is completed, the amount of antigens transferred from the fetus to the mother's body increases along with the course of pregnancy. Therefore, the pathogenic antibody production amount of the mother's body against the fetus also increases, and deterioration of the fetal condition is observed in proportion to the pathogenic antibody production amount and is accelerated especially after the second trimester of pregnancy.

### Blood type incompatibility

For example, a pregnancy in which the mother's body is Rho (D) negative while the fetus is Rho (D) positive is considered to be blood type incompatible pregnancy. After construction of the placenta, a small amount of fetal antigens including blood cell components migrate from the fetus to the mother's body via the placenta. At this time, a pathogenic antibody is produced by the mother's body against an antigen in which the migrated fetal antigen exceeds the immunological tolerance capacity of the mother's body, or an antigen that is not tolerated, and the pathogenic antibody migrates to the fetus via the placenta similarly to other antibodies and attacks a fetal antigen on the cell membrane or inside and outside the cells of the fetus as a target. In blood type incompatible pregnancy, an anti-D antibody produced by the mother's body migrates to the fetus via the placenta, destroys red blood cells in the fetal blood, and causes fetal anemia and later severe hydrops fetalis or intrauterine fetal death [Reference Literature 4]. This pathologic condition is not prominent in the first pregnancy, and after the second pregnancy, the pathologic condition becomes more severe as every time the chances of sensitization increase. Furthermore, after the placenta is constructed, since the amount of migrated antigens gradually increases along with the course of pregnancy, the pathologic condition progresses with the number of weeks of pregnancy. Particularly, the pathologic condition often deteriorates rapidly after the middle period in which the amount of migration becomes large. A treatment that is currently known is to perform plasmapheresis for removing an anti-D antibody produced by the mother's body, or fetal transfusion for anemia-stricken fetus, and promoting delivery in a state with low risk. Alternatively, in order to prevent sensitization to a D antigen, Rh-incompatible pregnant women must be treated with RhD immunoglobulin (Ig) during pregnancy, and in a case where a patient has not yet been sensitized, the patient should be treated after delivery or abortion. Since this is incompatibility between the mother's body and the fetus not only for RhD but also for other blood types, there is a possibility that blood type incompatible pregnancy may occur.

A mother having an anti-D antibody needs a management based on Doppler ultrasonography in order to measure the maternal blood antibody titers and the fetal middle cerebral artery (MCA) blood flow velocity, which is a predictive index for fetal anemia during pregnancy [Reference Literature 5].

On the other hand, fetal hemochromatosis is a disease that causes severe liver failure in the fetal period and the neonatal period, and a cause for the disease is presumed to be allogeneic immune fetal liver disorder. Although no pathogenic antigen has been identified, the disease develops when pregnancy occurs in a situation in which proteins (enzymes and the like) related to fetal iron metabolism are different from those of the mother's body.

After pregnancy, after construction of the placenta is completed, fetal proteins related to iron metabolism migrate to the mother's body via the placenta, and pathogenic antibodies are produced in the mother's body as a result of maternal immune response to the migrated fetal protein antigens. Fetal iron metabolism disorder, which occurs when pathogenic antibodies produced in the mother's body migrate to the fetus via the placenta and those pathogenic antibodies attack fetal proteins related to iron metabolism in the fetal body, is a basic pathogenic condition.

The recurrence rate of fetal hemochromatosis from the same mother is 90%, and regarding the treatment, combined therapy of internal medical treatment using iron chelating agents and antioxidants with liver transplantation has been conducted from around 1990; however, the survival rate of the infant was about 50% at the highest. In 2009, a therapeutic method based on postnatal fetal exchange transfusion and large-quantity γ-globulin therapy was reported as a new treatment method, and the survival rate of infants improved to 75%.

The current treatment method for fetal hemochromatosis involves subjecting a mother's body during pregnancy to high-dose γ-globulin therapy and preventing the onset of fetal hemochromatosis. However, since a large amount of γ-globulin is required, there is a further demand for the therapeutic method.

### Citation List

### Patent Literature

Patent Literature 1: WO 2016/068208 A

### Summary of Invention

### Technical Problem

It is an object of the present invention to provide a medicine for treating or ameliorating a disease associated with humoral immunity in the materno-fetal relationship, for example, sterility and infertility caused by humoral immunity in the materno-fetal relationship, blood type incompatible pregnancy, or fetal hemochromatosis.

### Solution to Problem

That is, in order to solve the above-described problems, the present invention includes the following embodiments.

### (Embodiment 1)

A medicine for treating a disease associated with humoral immunity in the materno-fetal relationship, the medicine including, as an active ingredient, a compound selected from the group consisting of:
(i) a compound represented by Formula (I) or a pharmaceutically acceptable salt thereof, 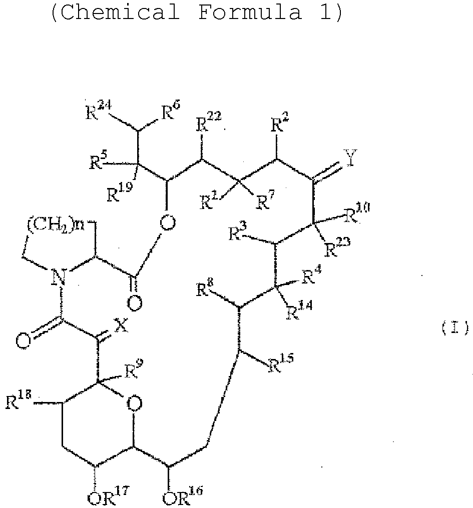 wherein each of the adjacent pairs of R¹ with R², R³ with R⁴, and R⁵ with R⁶ each independently
   (a) represents two adjacent hydrogen atoms, or R² may be an alkyl group, or
   (b) may form another bond between the carbon atoms to which the pair members are respectively bonded;
   R⁷ represents a hydrogen atom, a hydroxy group, or a protected hydroxy group, or may be bonded to R¹ and together represent an oxo group;
   R⁸ and R⁹ independently represent a hydrogen atom or a hydroxy group;
   R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
   X represents an oxo group, (a hydrogen atom, a hydroxy group), (a hydrogen atom, a hydrogen atom), or a group represented by formula: -CH₂O-;
   Y represents an oxo group, (a hydrogen atom, a hydroxy group), (a hydrogen atom, a hydrogen atom), or a group represented by formula: N-NR¹¹R¹² or N-OR¹³;
   R¹¹ and R¹² independently represent a hydrogen atom, an alkyl group, an aryl group, or a tosyl group;
   R¹³, R¹⁴, R¹⁵, R¹⁶, R¹⁷, R¹⁸, R¹⁹, R²², and R²³ independently represent a hydrogen atom or an alkyl group;
   R²⁴ represents a ring which can include one or more heteroatoms and may be substituted as desired;
   n represents 1 or 2; and
   in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof.

### (Embodiment 2)

The medicine according to Embodiment 1, wherein the compound represented by Formula (I) is tacrolimus or a pharmaceutically acceptable salt thereof.

### (Embodiment 3)

The medicine according to Embodiment 1, wherein the active ingredient is a compound represented by Formula (I) or a pharmaceutically acceptable salt thereof, and the compound represented by Formula (I) is tacrolimus or a pharmaceutically acceptable salt thereof.

### (Embodiment 4)

The medicine according to any one of Embodiments 1 to 3, wherein the disease associated with humoral immunity in the materno-fetal relationship is sterility and infertility caused by humoral immunity in the materno-fetal relationship.

### (Embodiment 5)

The medicine according to any one of Embodiments 1 to 3, wherein the disease associated with humoral immunity in the materno-fetal relationship is blood type incompatible pregnancy.

### (Embodiment 6)

The medicine according to any one of Embodiments 1 to 3, wherein the disease associated with humoral immunity in the materno-fetal relationship is fetal hemochromatosis.

### (Embodiment 7)

The medicine according to any one of Embodiments 4 to 6, wherein the medicine is applied to pregnancies of the second and succeeding children.

### (Embodiment 8)

The medicine according to any one of Embodiments 4 to 7, wherein the medicine is administered from early stage of pregnancy.

### (Embodiment 9)

The medicine according to any one of Embodiments 4 to 7, wherein the medicine is administered at a dose of 1 to 10 mg/day from the early stage of pregnancy.

### (Embodiment 10)

The medicine according to Embodiment 9, wherein the medicine is administered at a dose of 3 to 6 mg/day from the early stage of pregnancy.

### (Embodiment 11)

The medicine according to any one of Embodiments 1 to 3, 5, and 7 to 9, wherein the medicine is administered to a patient having a potential for blood type incompatible pregnancy in an amount of administration of 1 to 10 mg/day from the early stage of pregnancy.

Furthermore, the present invention also includes the following embodiments.

### (Embodiment 12)

A therapeutic method for a disease associated with humoral immunity in the materno-fetal relationship, the method including administering, to a patient, a compound selected from the group consisting of:
a compound represented by Formula (I) or a pharmaceutically acceptable salt thereof,
(ii) a cyclosporine, and
(iii) rapamycin or a derivative thereof.

### (Embodiment 13)

The therapeutic method according to Embodiment 12, the method including administering tacrolimus or a pharmaceutically acceptable salt thereof to a patient.

### (Embodiment 14)

The therapeutic method according to Embodiment 12 or 13, wherein the disease associated with humoral immunity in the materno-fetal relationship is blood type incompatible pregnancy.

### (Embodiment 15)

The therapeutic method according to Embodiment 12 or 13, wherein the disease associated with humoral immunity in the materno-fetal relationship is fetal hemochromatosis.

### (Embodiment 16)

The therapeutic method according to Embodiment 14 or 15, wherein the method is applied to pregnancies of the second and succeeding children.

### (Embodiment 17)

The therapeutic method according to any one of Embodiments 14 to 16, wherein administering is performed from the early stage of pregnancy.

### (Embodiment 18)

The therapeutic method according to any one of Embodiments 14 to 16, wherein administering is performed at a dose of 1 to 10 mg/day from the early stage of pregnancy.

### (Embodiment 19)

The therapeutic method according to any one of Embodiments 14 and 16 to 18, wherein a compound represented by Formula (I) is administered to a patient having a potential for blood type incompatible pregnancy in an amount of administration of 1 to 10 mg/day from the early stage of pregnancy.

### (Embodiment 20)

A compound for treating a disease associated with humoral immunity in the materno-fetal relationship, the compound being selected from the group consisting of:
a compound represented by Formula (I) or a pharmaceutically acceptable salt thereof,
(ii) a cyclosporine, and
(iii) rapamycin or a derivative thereof.

### (Embodiment 21)

Tacrolimus or a pharmaceutically acceptable salt thereof, for treating a disease associated with humoral immunity in the materno-fetal relationship.

### (Embodiment 22)

The compound according to Embodiment 20 or 21 or a pharmaceutically acceptable salt thereof, wherein the disease associated with humoral immunity in the materno-fetal relationship is blood type incompatible pregnancy.

### (Embodiment 23)

The compound according to Embodiment 20 or 21 or a pharmaceutically acceptable salt thereof, wherein the disease associated with humoral immunity in the materno-fetal relationship is fetal hemochromatosis.

### Advantageous Effects of Invention

According to the present invention, it is possible to treat or ameliorate a disease associated with humoral immunity in the materno-fetal relationship, for example, blood type incompatible pregnancy or fetal hemochromatosis.

### Brief Description of Drawings

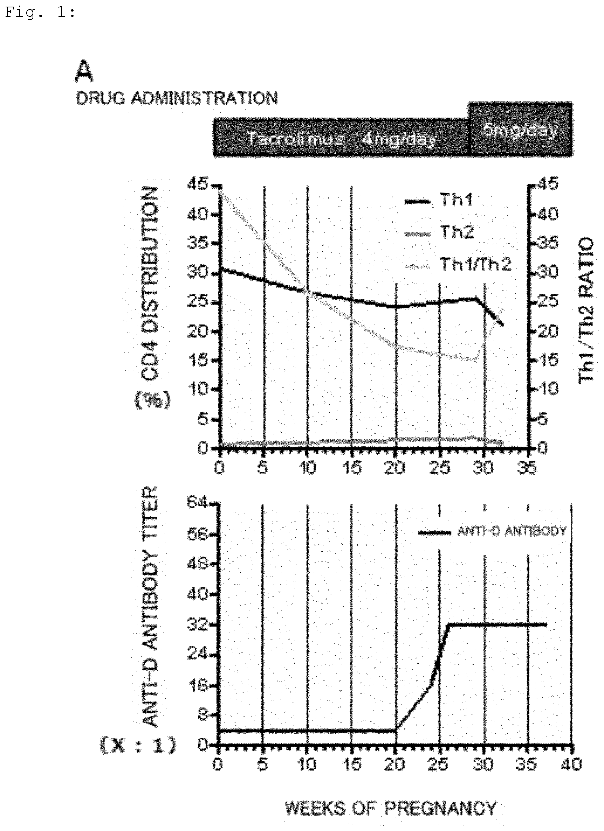

Fig. 1 shows, in the upper part, a graph showing Th1 and Th2 of a patient, and the ratio of the values. The initially high ratio of Th1/Th2 suggests that the patient suffers from infecundity caused by abnormality of the immune system. Fig. 1 shows, in the lower part, a graph showing the titer of an anti-D antibody of a pregnant woman for the weeks of pregnancy. It can be seen that the increase in the antibody titer caused by administration of tacrolimus in the early stage of pregnancy was gentle; however, when the amount of administration was increased (5 mg/day) in week 28 of pregnancy, where the antibody titer started to rapidly increase, the titer did not further increase and was stabilized.

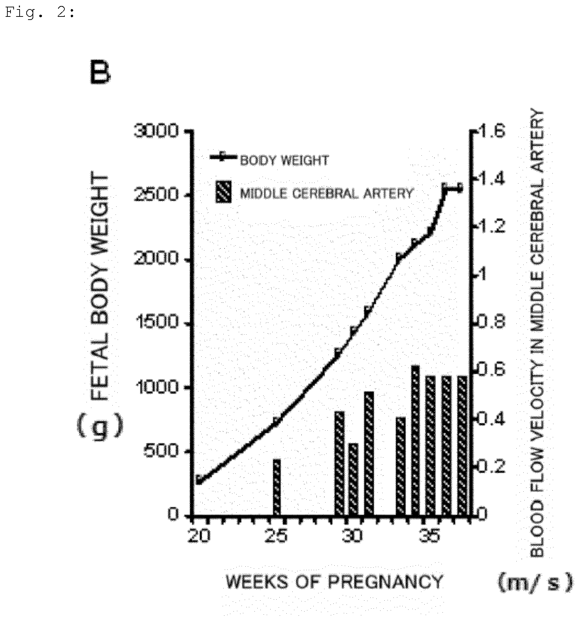

Fig. 2 is a graph showing the body weight of a fetus and the blood flow velocity in the middle cerebral artery in the weeks of pregnancy. The body weight smoothly increased from the early stage of pregnancy until even after week 28 where an increase in the administration of tacrolimus (5 mg/day) was required, the blood flow velocity serving as a measure for fetal anemia corresponded to the number of weeks.

### Description of Embodiments

Hereinafter, the present invention will be described in detail.

That is, the present invention provides a medicine for treating a disease associated with humoral immunity in the materno-fetal relationship, the medicine including a compound selected from the group consisting of:
(i) a compound represented by Formula (I) or a pharmaceutically acceptable salt thereof, 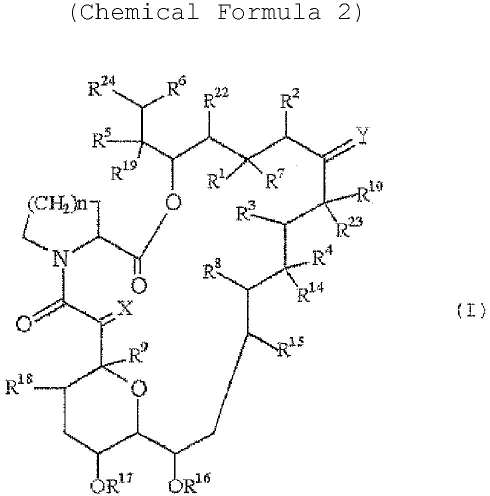 wherein each of the adjacent pairs of R¹ with R², R³ with R⁴, and R⁵ with R⁶ each independently:
   (a) represent two adjacent hydrogen atoms, or R² may be an alkyl group, or
   (b) may form another bond between the carbon atoms to which the pair members are respectively bonded (that is, forms a double bond);
   R⁷ represents a hydrogen atom, a hydroxy group, or a protected hydroxy group, or may be bonded to R¹ and together represent an oxo group;
   R⁸ and R⁹ independently represent a hydrogen atom or a hydroxy group;
   R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
   X represents an oxo group, (a hydrogen atom, a hydroxy group), (a hydrogen atom, a hydrogen atom), or a group represented by formula: -CH₂O-;
   Y represents an oxo group, (a hydrogen atom, a hydroxy group), (a hydrogen atom, a hydrogen atom), or a group represented by formula: N-NR¹¹R¹² or N-OR¹³;
   R¹¹ and R¹² independently represent a hydrogen atom, an alkyl group, an aryl group, or a tosyl group;
   R¹³, R¹⁴, R¹⁵, R¹⁶, R¹⁷, R¹⁸, R¹⁹, R²², and R²³ independently represent a hydrogen atom or an alkyl group;
   R²⁴ represents a ring which can include one or more heteroatoms and may be substituted as desired;
   n represents 1 or 2; and
   in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof, as an active ingredient.

In the compound represented by Formula (I), R²⁴ represents a ring that can include one or more heteroatoms and may be substituted as desired, and specifically represents a 5-membered to 7-membered carbocyclic ring or a 5-membered or 6-membered heterocyclic group. An example of the 5-membered or 6-membered heterocyclic group may be a saturated or unsaturated, 5-membered or 6-membered heterocyclic group containing one or more heteroatoms selected from a nitrogen atom, a sulfur atom, and an oxygen atom. A preferred example of R²⁴ may be a cyclo- (C₅-C₇) alkyl group which may have an appropriate substituent, and examples thereof include, for example, the following groups:
(a) a 3,4-dioxo-cyclohexyl group;
(b) a 3-R²⁰-4-R²¹-cyclohexyl group
   [wherein R²⁰ represents a hydroxy, an alkyloxy, an oxo, or OCH₂OCH₂CH₂OCH₃, and R²¹ represents a hydroxy, -OCN, an alkyloxy, a heteroaryloxy which may have an appropriate substituent, -OCH₂OCH₂CH₂OCH₃, a protected hydroxy, a chloro, a bromo, an iodo, an aminooxalyloxy, an azide group, a p-tolyloxythiocarbonyloxy, or R²⁵R²⁶CHCOO- (wherein R²⁵ represents a hydroxy group which may be protected as desired, or a protected amino group; and R²⁶ represents a hydrogen atom or a methyl), or R²⁰ and R²¹ may be bonded together and form an oxygen atom of an epoxide ring (that is, -O-)]; or
(c) a cyclopentyl group, the cyclopentyl group possibly being substituted with a methoxymethyl, a hydroxymethyl protected as desired, an acyloxymethyl (wherein the acyl moiety is a dimethylamino group which may be quaternized as desired, or a carboxyl group which may be esterified as desired), one or more amino and/or hydroxy groups which may be protected, or an aminooxalyloxymethyl, while a preferred example is a 2-formylcyclopentyl group.

Various definitions and specific examples thereof as used in the present specification, and preferred embodiments thereof will be described in detail below.

Unless particularly stated otherwise, the term "lower" is intended to mean a group having 1 to 6 carbon atoms.

A preferred example of the alkyl moiety of the "alkyl group" and the "alkyloxy group" may be a linear or branched aliphatic hydrocarbon residue, and examples include lower alkyl groups having 1 to 6 carbon atoms, such as methyl, ethyl, propyl, isopropyl, butyl, isobutyl, pentyl, neopentyl, and hexyl.

A preferred example of the "alkenyl group" may be a linear or branched aliphatic hydrocarbon residue containing one double bond, and examples include lower alkenyl groups such as vinyl, propenyl (allyl or the like), butenyl, methylpropenyl, pentenyl, and hexenyl.

Preferred examples of the "aryl group" include phenyl, tolyl, xylyl, cumenyl, mesityl, and naphthyl.

Examples of a preferred protective group for the "protected hydroxy group" and the "protected amino" include 1-(lower alkylthio) (lower) alkyl groups such as, for example, lower alkylthiomethyl groups such as methylthiomethyl, ethylthiomethyl, propylthiomethyl, isopropylthiomethyl, butylthiomethyl, isobutylthiomethyl, or hexylthiomethyl, with a more preferred example being a C₁-C₄ alkylthiomethyl group, and the most preferred example being a methylthiomethyl group;
trisubstituted silyl groups, for example, a tri(lower) alkylsilyl such as trimethylsilyl, triethylsilyl, tributylsilyl, tertiary butyldimethylsilyl, or tri-tertiary butylsilyl, and for example, a lower alkyldiarylsilyl such as methyldiphenylsilyl, ethyldiphenylsilyl, propyldiphenylsilyl, or tertiary butyldiphenylsilyl, with more preferred examples being a tri(C₁-C₄) alkylsilyl group and a C₁-C₄ alkyldiphenylsilyl group, and the most preferred examples being a tertiary butyldimethylsilyl group and a tertiary butyldiphenylsilyl group; and
acyl groups such as an aliphatic acyl group derived from a carboxylic acid, a sulfonic acid or a carbamic acid, an aromatic acyl group, and an aliphatic acyl group substituted with an aromatic group.

Examples of the aliphatic acyl group include a lower alkanoyl group which may have one or more appropriate substituents such as a carboxyl, for example, formyl, acetyl, propionyl, butyryl, isobutyryl, valeryl, isovaleryl, pivaloyl, hexanoyl, carboxyacetyl, carboxypropionyl, carboxybutyryl, or carboxyhexanoyl;
a cyclo-(lower) alkyloxy-(lower) alkanoyl group which may have one or more appropriate substituents such as a lower alkyl, for example, cyclopropyloxyacetyl, cyclobutyloxypropionyl, cycloheptyloxybutyryl, menthyloxyacetyl, menthyloxypropionyl, menthyloxybutyryl, menthyloxypentanoyl, or menthyloxyhexanoyl;
a camphorsulfonyl group; and
a lower alkylcarbamoyl group having one or more appropriate substituents such as a carboxyl or a protected carboxyl, for example, a carboxy-(lower) alkylcarbamoyl group such as carboxymethylcarbamoyl, carboxyethylcarbamoyl, carboxypropylcarbamoyl, carboxybutylcarbamoyl, carboxypentylcarbamoyl, or carboxyhexylcarbamoyl, or a tri-(lower) alkylsilyl-(lower) alkyloxycarbonyl-(lower) alkylcarbamoyl group such as trimethylsilylmethoxycarbonylethylcarbamoyl, trimethylsilylethoxycarbonylpropylcarbamoyl, triethylsilylethoxycarbonylpropylcarbamoyl, tertiary butyldimethylsilylethoxycarbonylpropylcarbamoyl, or trimethylsilylpropoxycarbonylbutylcarbamoyl group.

Examples of the aromatic acyl group include an aroyl group which may have one or more appropriate substituents such as a nitro, for example, benzoyl, toluoyl, xyloyl, naphthoyl, nitrobenzoyl, dinitrobenzoyl, or nitronaphthoyl; and
an arenesulfonyl group which may have one or more appropriate substituents such as a halogen, for example, benzenesulfonyl, toluenesulfonyl, xylenesulfonyl, naphthalenesulfonyl, fluorobenzenesulfonyl, chlorobenzenesulfonyl, bromobenzenesulfonyl, or iodobenzenesulfonyl.

Examples of the aliphatic acyl group substituted with an aromatic group include an aryl-(lower) alkanoyl group which may have one or more appropriate substituents such as a lower alkyloxy or a trihalo-(lower) alkyl, for example, phenylacetyl, phenylpropionyl, phenylbutyryl, 2-trifluoromethyl-2-methoxy-2-phenylacetyl, 2-ethyl-2-trifluoromethyl-2-phenylacetyl, or 2-trifluoromethyl-2-propoxy-2-phenylacetyl.

Among the acyl groups described above, more preferred examples of the acyl group include a C₁-C₄ alkanoyl group which may have a carboxyl, a cyclo-(C₅-C₆) alkyloxy- (C₁-C₄) alkanoyl group having two (C₁-C₄) alkyls in the cycloalkyl moiety, a camphorsulfonyl group, a carboxy-(C₁-C₄) alkylcarbamoyl group, a tri- (C₁-C₄) alkylsilyl- (C₁-C₄) alkyloxycarbonyl-(C₁-C₄) alkylcarbamoyl group, a benzoyl group which may have one or two nitro groups, a benzenesulfonyl group having a halogen, and a phenyl-(C₁-C₄) alkanoyl group having a C₁-C₄ alkyloxytrihalo- (C₁-C₄) alkyl, and among them, most preferred examples include acetyl, carboxypropionyl, menthyloxyacetyl, camphorsulfonyl, benzoyl, nitrobenzoyl, dinitrobenzoyl, iodobenzenesulfonyl, and 2-trifluoromethyl-2-methoxy-2-phenylacetyl.

Examples of the "5-membered to 7-membered carbocyclic ring" include 5-membered to 7-membered cycloalkyl groups or cycloalkenyl groups, and examples thereof include cyclopentyl, cyclohexyl, cycloheptyl, cyclopentenyl, cyclohexenyl, and cycloheptenyl.

Preferred examples of the "heterocyclic group having a saturated or unsaturated, 5-membered or 6-membered ring and containing one or more heteroatoms selected from a nitrogen atom, a sulfur atom, and an oxygen atom" include a pyrrolyl group and a tetrahydrofuryl group.

Examples of the "heteroaryl moiety which may have an appropriate substituent" in the "heteroaryloxy which may have an appropriate substituent" include the moieties listed as examples of group R¹ of the compound represented by the formula shown in EP-A-532,088, and for example, 1-hydroxyethylindol-5-yl is preferred. The disclosure of the patent literature is partially incorporated herein by reference.

### Active ingredient

According to the present invention, (i) a compound represented by Formula (I) or a pharmaceutically acceptable salt thereof, (ii) a cyclosporine, or (iii) rapamycin or a derivative thereof can be used as an active ingredient. Furthermore, two or more kinds of (i) a compound represented by Formula (I), (ii) a cyclosporine, or (iii) rapamycin or a derivative thereof may also be used in combination as the active ingredient. The respective active ingredients will be described below.

### (i) Compound represented by Formula (I)

The compound represented by Formula (I) or a pharmaceutically acceptable salt thereof, which is used for the present invention, is as described above, and specifically, the compound or the salt is described in, for example, EP-A-184162, EP-A-323042, EP-A-423714, EP-A-427680, EP-A-465426, EP-A-480623, EP-A-532088, EP-A-532089, EP-A-569337, EP-A-626385, WO 89/05303, WO 93/05058, WO 96/31514, WO 91/13889, WO 91/19495, and WO 93/5059.

Particularly, compounds called FR900506 (= FK506, tacrolimus), FR900520 (ascomycin), FR900523, and FR900525 are substances produced by the genus Streptomyces, for example, Streptomyces tsukubaensis No. 9993 (depository: National Institute of Bioscience and Human-Technology Agency of Industrial Science and Technology, the Ministry of International Trade and Industry, 1-3, Higashi 1-chome, Tsukuba-shi, Ibaraki-ken, Japan (formerly: Fermentation Research Institute, Agency of Industry Science and Technology, the Ministry of International Trade and Industry), date of deposit: Oct. 5, 1984, deposit number: FERM BP-927) or Streptomyces hygroscopicus subsp. yakushimaensis No. 7238 (depository: National Institute of Bioscience and Human-Technology Agency of Industrial Science and Technology, the Ministry of International Trade and Industry, 1-3, Higashi 1-chome, Tsukuba-shi, Ibaraki-ken, Japan, date of deposit: Jan. 12, 1985, deposit number: FERM BP-928) (EP-A-0184162), and particularly, FK506 (general name: tacrolimus) represented by the following structural formula is a representative compound.

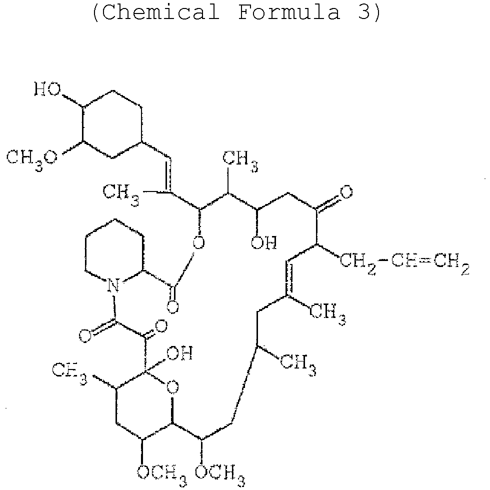

Chemical name: 17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.04,9]octacos-18-ene-2,3,10,16-tetraone.

As a particularly preferred embodiment, the compound represented by Formula (I) is such that each of the adjacent pairs of R³ with R⁴ and R⁵ with R⁶ forms another bond between the carbon atoms to which the pair members are respectively bonded (thus, a double bond is formed at the moieties of R³ with R⁴ and R⁵ with R⁶) ;
R¹, R², R⁸, and R²³ independently represent a hydrogen atom;
R⁹ represents a hydroxy group; R¹⁰ represents a methyl, ethyl, propyl, or allyl group;
R⁷ represents a hydroxy;
X represents (a hydrogen atom, a hydrogen atom) or an oxo group;
Y represents an oxo group;
R¹⁴, R¹⁵, R¹⁶, R¹⁷, R¹⁸, R¹⁹, and R²² each represent a methyl group; and
R²⁴ represents a 3-R²⁰-4-R²¹-cyclohexyl group,
wherein R²⁰ represents a hydroxy, an alkyloxy, an oxo, or -OCH₂OCH₂CH₂OCH₃; and
R²¹ represents a hydroxy, -OCN, an alkyloxy, a heteroaryloxy which may have an appropriate substituent, a 1-tetrazolyl or a 2-tetrazolyl, -OCH₂OCH₂CH₂OCH₃, a protected hydroxy, a chloro, a bromo, an iodo, an aminooxalyloxy, an azide group, a p-tolyloxythiocarbonyloxy, or R²⁵R²⁶CHCOO- (wherein R²⁵ represents a hydroxy group which may be protected as desired, or a protected amino group; and R²⁶ represents a hydrogen atom or a methyl), or
R²⁰ and R²¹ may be bonded together and form an oxygen atom of an epoxide ring (that is, -O-); and n represents 1 or 2.

Other preferred embodiments of the compound represented by Formula (I) include tacrolimus, and ascomycin or a derivative thereof.

Furthermore, the compounds described in EP 0184162, EP 323042, EP 424714, EP 427680, EP 465426, EP 474126, EP 480623, EP 484936, EP 532088, EP 532089, EP 569337, EP 626385, WO 89/05303, WO 93/05058, WO 96/31514, WO 91/13889, WO 91/19495, WO 93/5059, WO 96/31514, and the like may also be listed as preferred examples of the compound represented by Formula (I) of the present invention, the disclosures of which are partially incorporated herein by reference.

### Pharmaceutically acceptable salt of compound represented by Formula (I)

The term "pharmaceutically acceptable salt" for the compound represented by Formula (I) of the present invention refers to a salt prepared from a pharmaceutically acceptable, non-toxic base or acid. In a case where the compound represented by Formula (I) of the present invention is acidic, a salt corresponding thereto can be conveniently prepared from a pharmaceutically acceptable non-toxic base, which includes an inorganic base and an organic base. Examples of a salt derived from such an inorganic base include salts of aluminum, ammonium, calcium, copper (cupric and cuprous), ferric, ferrous, lithium, magnesium, manganese (manganic and manganous), potassium, sodium, and zinc. Salts of ammonium, calcium, magnesium, potassium, and sodium are preferred. The salt prepared from a pharmaceutically acceptable non-toxic organic base includes salts of primary, secondary, and tertiary amines derived from both naturally occurring and synthetic sources. Examples of a pharmaceutically acceptable non-toxic organic base include arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, dicyclohexylamine, lysine, methylglucamine, morpholine, piperazine, piperidine, a polyamine resin, procaine, purine, theobromine, triethylamine, trimethylamine, tripropylamine, and tromethamine.

In a case where the compound represented by Formula (I) of the present invention is basic, a salt corresponding thereto can be conveniently prepared from a pharmaceutically acceptable non-toxic base, which includes an inorganic acid and an organic acid. Examples of such an acid include acetic acid, benzenesulfonic acid, benzoic acid, camphorsulfonic acid, citric acid, ethanesulfonic acid, fumaric acid, gluconic acid, glutamic acid, hydrobromic acid, hydrochloric acid, isethionic acid, lactic acid, maleic acid, malic acid, mandelic acid, methanesulfonic acid, muconic acid, nitric acid, pamoic acid, pantothenic acid, phosphoric acid, succinic acid, sulfuric acid, tartaric acid, and p-toluenesulfonic acid. Citric acid, hydrobromic acid, hydrochloric acid, maleic acid, phosphoric acid, sulfuric acid, and tartaric acid are preferred.

### Crystal form of compound represented by Formula (I)

The compound represented by Formula (I) of the present invention can exist in an amorphous form and/or in one or more crystalline forms, and all such amorphous form and crystalline forms of the compound represented by Formula (I) as well as a mixture of those are intended to be included in the scope of the present invention. Furthermore, some of the compounds represented by Formula (I) can form solvates with water (that is, hydrates) or solvates with conventional organic solvents. Such solvates and hydrates, particularly pharmaceutically acceptable solvates and hydrates, of the compounds represented by Formula (I) are likewise included in the scope of the compound defined by Formula (I) and pharmaceutically acceptable salts thereof, together with unsolvated anhydrous forms of the compound.

### Isomers of compound represented by Formula (I)

With regard to the compound represented by Formula (I) of the present invention, there may be one or more pairs of steric isomers such as a conformer or an optical isomer attributable to an asymmetric carbon atom and a double bond and a geometrical isomer, and such conformer or isomers are also included in the scope of the compounds of the present invention.

### Solvate or hydrate of compound represented by Formula (I)

The compound represented by Formula (I) of the present invention may form a solvate, and that case is also included in the scope of the present invention. Preferred examples of the solvate include a hydrate and an ethanolate.

### Method for preparing compound represented by Formula (I)

The compound represented by Formula (I) of the present invention is a compound known in the cited references, and a production method for the compound is disclosed in, for example, EP-A-184162, EP-A-323042, EP-A-423714, EP-A-427680, EP-A-465426, EP-A-480623, EP-A-532088, EP-A-532089, EP-A-569337, EP-A-626385, WO 89/05303, WO 93/05058, WO 96/31514, WO 91/13889, WO 91/19495, and WO 93/5059. Furthermore, tacrolimus is marketed from Astellas Pharma, Inc. under the brand name of PROGRAF (registered trademark).

### (ii) Cyclosporine

Examples of the cyclosporine include cyclosporines A, B, and D, and these are described in Merck Index (12th edition) No. 2821. Furthermore, cyclosporines are marketed from Novartis Pharma AG under the brand name of SANDIMMUNE.

### (iii) Rapamycin derivative

Rapamycin (also called sirolimus) is described in Merck Index (12th edition) No. 8288, and derivatives thereof can also be used. Preferred examples include O-substituted derivatives in which the hydroxy at the 40-position of Formula A in page 1 of WO 95/16691 is substituted by -OR¹ (wherein R¹ represents a hydroxyalkyl, a hydroalkyloxyalkyl, an acylaminoalkyl, and an aminoalkyl), for example, 40-O-(2-hydroxy)ethyl-rapamycin, 40-O-(3-hydroxy)propyl-rapamycin, 40-O-[2-(2-hydroxy)ethoxy]ethyl-rapamycin, and 40-O-(2-acetaminoethyl)-rapamycin. Furthermore, rapamycin (sirolimus) is marketed from Nobelpharma Co., Ltd. under the brand name of RAPALIMUS.

The compound represented by Formula (I) of the present invention, a cyclosporine, rapamycin and a derivative thereof have a similar basic skeleton, that is, a tricyclomacrolide skeleton and have at least one similar biological characteristics (for example, immunosuppressive action) .

### Other optional components

The medicine according to the present invention may include, in addition to the above-described active ingredients, one or more therapeutically active substances having therapeutic action on other diseases, illnesses, and conditions, so long as the therapeutically active substances have no risk of inhibiting the activity of the active ingredient and are not harmful to the subject for administration (hereinafter, also referred to as patient).

### Medicine of present invention

The medicine of the present invention includes a compound selected from the group consisting of: (i) a compound represented by Formula (I) or a pharmaceutically acceptable salt thereof, (ii) a cyclosporine, and (iii) a rapamycin derivative as an active ingredient, and may further include a pharmaceutically acceptable carrier that is not harmful to the subject for administration. The carrier that can be used may be any of a solid type, a semisolid type, and a liquid type and may be, for example, any one selected from water, a liquid electrolyte, a glucose solution, and the like; however, the carrier is not limited to these. Furthermore, the medicine may also include auxiliary agents. Examples of the auxiliary agents include a lubricating agent, a stabilizer, an antiseptic agent, an emulsifier, a thickener (viscous agent), a colorant, a fragrance (flavoring agent), an excipient, a preservative, a buffering agent, a corrigent, a suspending agent, an emulsifier, and a dissolution aid.

### Dosage form

The medicine including the active ingredient of the present invention can be provided in various dosage forms, and examples of the dosage forms include a tablet, a capsule, a pill, a granular preparation, a powder, a syrup, a suppository, a troche, a pellet, an emulsion, a suspension, and other known forms. Among these, for example, the dosage form as a preparation for oral administration is preferably any of a tablet, a capsule, a pill, a granular preparation, a powder, a liquid, a syrup, and a jelly; more preferably any of a tablet, a capsule, and a granular preparation; and even more preferably a tablet. In addition, as will be described below, the medicine may be formulated as a preparation for parenteral administration such as, for example, an injectable preparation, a suppository, and a percutaneous absorption type preparation.

### Method for producing medicine

The medicine according to the present invention can be produced by utilizing any known production method. For example, the medicine is produced by producing an active ingredient and other optional components separately for each component, and then mixing the respective components so as to obtain desired contents.

### Subject for administration of medicine

Examples of the subject for administration of the medicine of the present invention include mammals. Examples of the mammals include human being and non-human animals, including domestic animals such as cow, horse, pig, and sheep; monkey, chimpanzee; and pets such as dog, cat, rat, and rabbit, and a preferred mammal is human being.

### Route of administration

The method for administration (route of administration) of the medicine of the present invention can be appropriately determined based on the age and condition of the subject for administration, the duration of treatment, and the like. Specifically, both oral administration and parenteral administration may be employed; however, oral administration is preferred (oral administration is employed in Examples). Examples of parenteral administration include methods such as administration by injection, administration using a suppository, and administration using a percutaneous absorption type preparation. Examples of the type of administration by injection include intramuscular injection, intraperitoneal injection, subcutaneous injection, intravenous injection, and topical injection. Furthermore, the medicine of the present invention can be administered through various routes such as percutaneous, transnasal, transvaginal, and transrectal routes.

### Amount of administration

The amount of administration of the medicine varies depending on the type of the disease, illness or condition of the patient who receives administration of the medicine, severity, results of various examinations, the type of the active ingredient of the medicine, and the like. Furthermore, the amount of administration of the medicine also varies depending on the age of the patient to be treated, the number of times of treatment according to the treatment method of the present invention, results of various examinations, and the like. For instance, from the viewpoint of the content of the active ingredient included in the medicine, the medicine of the present invention is administered at a dose that is lower than the amount of administration in a case where the medicine is used as an immunosuppressant in living donor organ transplantation, the treatment for immune system diseases, and the like. For example, in a case where the subject for administration of the medicine is a human being, without being particularly limited, the medicine is administered in an amount, as an amount of the active ingredient, preferably in the range of 0.5 to 10 mg or 1 to 10 mg, and more preferably in the range of 0.5 to 6 mg or 3 to 6 mg, per day. In addition, hereinafter, unless particularly stated otherwise, the description concerning the amount of administration of the medicine is applied in a case where the subject is a human being, and the amount of administration is indicated as the amount of the active ingredient.

Furthermore, without being particularly limited, in the case of oral administration, the frequency of administration per day is preferably 1 to 4 times, more preferably 1 to 3 times, and even more preferably 1 to 2 times.

In a case where the compound as the active ingredient of the present invention is administered stepwise or in combination with another therapeutic drug, generally the same dosage form can be used. In a case where drugs are physically combined and administered, the dosage form and the route of administration should be selected according to the compatibility of the drugs to be combined. Therefore, the term simultaneous administration is understood to include simultaneous or continuous administration of two medicines, or administration of two active ingredients as a combination at fixed doses.

### Sterility and infertility caused by humoral immunity in materno-fetal relationship

The term "sterility and infertility" is a concept including both sterility and infertility. The term "sterility" according to the present specification refers, in a broad sense, to a state in which the mother's body has difficulties in becoming pregnant compared to a normal state, and the term is a concept including infecundity. The term "infecundity" according to the present specification refers to a case where pregnancy is not established within one year without contraception under an expectation of childbearing. The term "infertility" according to the present specification refers to a state in which after conception, the fetus in the womb of the mother's body is incapable of growing, or a state in which retarded growth or defective growth of the fetus is observed, and the term is a concept including infertilitas. The term "infertilitas" refers to a case where pregnancy (also including spontaneous pregnancy as well as the cases of artificial fertilization and in vitro fertilization) is established, but miscarriage, premature birth, or stillbirth is repeated once or two or more times so that childbearing is not achieved.

Here, the causes for sterility including infecundity include an ovum factor, an oviduct factor, a uterine factor, a cervical duct factor, and an immunological factor. On the other hand, the causes for infertility including infertilitas include a genetic factor, an anatomic factor, an endocrine factor, a coagulative factor, and an autoimmunologic factor.

The "sterility and infertility caused by humoral immunity in the materno-fetal relationship" according to the present specification includes only the sterility and infertility abnormally caused by humoral immunity in the materno-fetal relationship, particularly among "sterilities and infertilities". Therefore, in the "sterility and infertility caused by humoral immunity in the materno-fetal relationship" according to the present specification, "sterility and infertility" abnormally caused by cell-mediated immunity is not included.

The sterility and infertility caused by humoral immunity in the materno-fetal relationship are, for example, disorders occurring in the following cases.
(i) A case where sterility and infertility are not caused by any of an ovum factor, an oviduct factor, a uterine factor, a cervical duct factor, and an immunological factor (particularly a cell-mediated immunological factor), all of which are known as main causes of sterility.
(ii) A case where sterility and infertility are not caused by any of a genetic factor, an anatomic factor, an endocrine factor, a coagulative factor, and an autoimmunologic factor, all of which are known as causes of infertility.
(iii) A case where the cause is not known, and the parameters that identify abnormality of cell-mediated immunity, such as the Th1/Th2 cell ratio, show normal values (that is, a case where abnormality of cell-mediated immunity is not a cause).
(iv) A case where a pathogenic antibody targeting a fetal antigen on the cell membrane or inside and outside the cells of a fertilized ovum or a fetus exists in the mother's body, and the mother's body directly attacks the fertilized ovum or the fetal components in the uterus by means of the pathogenic antibody, or the pathogenic antibody that has migrated via the placenta attacks the fetus.

The amount of administration of the compound as the active ingredient of the present invention at this time is preferably 1 to 10 mg/day, and more preferably 3 to 6 mg/day.

### Blood type incompatible pregnancy

Blood type incompatible pregnancy is a disorder occurring in a state such as follows.
(i) An antigen that is absent on the red blood cell membrane of the mother's body exists on the red blood cell membrane of the fetus,
(ii) after the fetal blood flows into the mother's body via the placenta, a pathogenic antibody to an antigen on the fetal red blood cell membrane is produced in the mother's body (an increase in the pathogenic antibody, for example, the anti-D antibody titer, an anti-red blood cell antibody, and the like),
(iii) a pathogenic antibody migrates to the fetus via the placenta and attacks fetal red blood cells, and
(iv) the fetal red blood cells hemolyze and cause fetal anemia.

Therefore, by administering the medicine of the present invention (for example, tacrolimus) to a pregnant woman from before the increase in the pathogenic antibody titer as in (ii), for example, from immediately after conception, an increase in the pathogenic body titer is suppressed (suppression of production of the antibody), symptoms caused by blood type incompatible pregnancy are suppressed, and therefore, it is possible to carry out treatment or amelioration of blood type incompatible pregnancy.

An example of the amount of administration is 1 mg/day to 10 mg/day in terms of the active ingredient. Preferably, the amount of administration is 3 to 6 mg/day.

Then, regarding the attack of fetal red blood cells by a pathogenic antibody that has passed through the placenta as in (iii), since an antigen on the fetal red blood cell membrane is once (through pregnancy, miscarriage or the like of the previous time) recognized, and the memory for pathogenic antibody production remains, blood type incompatible pregnancy is more likely to occur at the time of a pregnancy of the second and succeeding children than at the time of a pregnancy of the first child.

Furthermore, in a case where a woman suffers from infecundity, and pregnancy has been established using, for example, the medicine of the present invention (1 to 4 mg/day in terms of the active ingredient), the medicine is continuously administered, and for example, when an increase in the pathogenic antibody titer is recognized, the medicine may be administered after the amount of the medicine is further increased (for example, 5 to 10 mg/day in terms of the active ingredient).

Furthermore, even in a case where a woman suffers from infecundity, and pregnancy has been established by, for example, a method other than the medicine of the present invention, treatment of blood type incompatible pregnancy using the medicine of the present invention is made possible, and for example, the medicine of the present invention (for example, 1 to 10 mg/day in terms of the active ingredient) may be administered from the early stage of pregnancy.

### Fetal hemochromatosis

Fetal hemochromatosis is a symptom occurring in a state such as follows.
(i) An enzyme related to iron metabolism of the fetus is different from that of the mother's body,
(ii) after fetal blood flows into the mother's body via the placenta, a pathogenic antibody to the enzyme is produced in the mother's body,
(iii) the pathogenic antibody migrates to the fetus via the placenta and attacks the iron metabolism enzyme of the fetus, and
(iv) iron metabolism of the fetus stops, and iron deposits in the liver to result in liver cirrhosis.

Therefore, by administering the medicine of the present invention (for example, tacrolimus) to a pregnant woman from before an increase in the pathogenic antibody titer as in (ii), for example, from immediately after conception, an increase in the pathogen body titer is suppressed (suppression of production of antibody), symptoms caused by the pathogenic antibody are suppressed, and therefore, it is possible to carry out treatment or amelioration of fetal hemochromatosis.

The amount of administration of the compound as the active ingredient of the present invention at this time is preferably 1 to 10 mg/day, and more preferably 3 to 6 mg/day.

### Examples

Hereinafter, the present invention will be described by way of specific embodiments; however, the present invention is not intended to be limited to those embodiments, and it should be understood that various alterations and modifications in those embodiments can be carried out by those ordinarily skilled in the art, without deviating from the scope or purport of the present invention as defined in the attached claims.

### Measurement of anti-D antibody titer

The measurement is carried out by the indirect Coombs test. The indirect Coombs test is a test in which an anti-immunoglobulin antibody is added to a mixture of the blood serum of a patient and the blood of a healthy person to examine whether a hemagglutination reaction occurs (irregular antibody present in the blood serum is detected) (for the Coombs test, see, for example, Nissan Women's Journal Vol. 59, No. 10, N-617-N-623).

### Th1/Th2 cell ratio

In recent years, the incidence of in vitro fertilization (IVF) and embryo transplantation (ET) is increasing all over the world. Along with this, the number of women experiencing multiple IVF failures, including repeated implantation failure, is increasing. Upon conducting IVF/ET, embryos are transplanted into the uterine cavity within 2 to 5 days after fertilization. Pregnancy is established when a so-called semi-allograft embryo is successfully implanted on the maternal decidua concomitantly with the establishment of immunological tolerance on the mother's body side [Reference Literature 7]. Establishing an appropriate immune response at the time of implantation is the key to successful implantation. Therefore, immunological etiology is considered to play an important role in RIF after IVF/ET.

T helper (Th)1, Th2, Th17, and Treg cells accomplish important roles in regard to immune responses such as, for example, immunological rejection and immunological tolerance [Reference Literature 8]. It is generally agreed that the immune status during pregnancy is associated with Th2 dominance and the Th1 immune response is associated with embryonic rejection [Reference Literatures 6 and 9]. The underlying mechanism of embryonic rejection is considered to be similar to a rejection reaction in allograft [Reference Literature 10]. Embryos transplanted during IVF/ET may fail to implant due to an immune response similar to a rejection reaction in allograft.

### Analysis of Th1 cells and Th2 cells

For the purpose of evaluating the baseline value of the Th1/Th2 cell ratio, a total of 10 ml of venous blood was collected. Th1 cells and Th2 cells were determined by detecting the production of intracellular interferon (IFN)-γ and IL-4.

Specific staining of lymphocytes was performed by incubating whole blood together with anti-CD4-PC5 or anti-CD8-PC5-conjugated monoclonal antibody (mAbs) (Beckman Coulter, Inc., Fullerton, Ca, USA). Red blood cells (RBCs) were removed by hemolysis (using FACS Lysing solution; Becton, Dickinson and Company, BD 134 Biosciences, Franklin Lake, NJ, USA), and lymphocytes were analyzed using flow cytometry (FACSCalibur; Becton, Dickinson and Company). Activated whole blood samples were surface-stained using anti-CD4-PC5-conjugate (Abs), and then RBC hemolysis and specific intracellular staining using FastImmune (trademark) IFN-γ-FITC/IL-4-PE (Becton, Dickinson and Company) were carried out in sequence according to the manufacturer's instructions for use. Th1 cells were defined as CD4⁺ lymphocytes accompanied by intracellular IFN-γ but not by intracellular IL-4. Th2 cells were detected as CD4⁺ lymphocytes accompanied by intracellular IL-4 but not by intracellular IFN-γ. The ratio of intracellular IFN-γ-positive Th cells with respect to intracellular IL-4-positive Th cells was expressed as the Th1/Th2 cell ratio.

### Example 1

### Treatment of blood type incompatible pregnancy with tacrolimus

The patient is a 35-year-old woman having blood type A. The patient suffering from a blood type incompatible pregnancy with negative maternal Rho (D) and positive fetal Rho (D) was subjected to the following treatments. That is, at the time of the first pregnancy, anti-D immunoglobulin was not administered during pregnancy, sensitization was effected before delivery, and the anti-D antibody titer at the time of delivery was 8-fold. In week 39, she gave birth by emergency cesarean section due to placental abruption. After delivery, the antibody titer obtained after 5 months showed a maximum value of 64-fold.

Two years later, she hoped to have a second child but suffered from infecundity and was treated for sterility by in vitro fertilization; however, since the treatment was unsuccessful after five embryo transplantations, a close examination of the immune system was conducted. At that time, a marked increase (24.9) in the Th1/Th2 (Th1 32.4, Th2 1.3) ratio was recognized, the illness was judged as infecundity considered to be caused by an abnormality in the immune system, and a tacrolimus treatment was selected. Pregnancy was established by a single embryo transplantation with a treatment using 4 mg/day of tacrolimus (oral administration: 2 mg in the morning, 2 mg in the evening).

The anti-D antibody titer obtained immediately after the establishment of pregnancy was 4-fold; however, the titer reached 16-fold in week 24 and reached 32-fold in week 26 (Fig. 1, upper part). After that, migration of fetal antigens to the mother's body via the placenta was assumed to further increase, and preparation of plasmapheresis and fetal blood transfusion was initiated in preparation for a rapid increase in the anti-D antibody titer. Furthermore, at the same time, the dose of tacrolimus was increased to 5 mg/day (oral administration: 3 mg in the morning and 2 mg in the evening), in consideration of the fact that Th1 in week 28 showed a tendency of increase again.

Since then, the expected increase in the anti-D antibody titer did not occur at all, the 32-fold was maintained without observing any fetal anemia (increased blood flow velocity in the middle cerebral artery), growth of the child occurred without any problem (Fig. 2), and the patient gave birth to a healthy boy who weighed 2834 g on day 2 of week 37. Furthermore, the blood flow velocity in the middle cerebral artery and the estimated body weight of the fetus were measured by fetal ultrasonography.

Fig. 2 is a graph showing the change in the body weight of a fetus during the weeks of pregnancy. The body weight is increasing over time, which shows that the fetus is growing correspondingly to the number of weeks of pregnancy. At the same time, Fig. 2 also shows the blood flow velocity in the middle cerebral artery and shows that fetal anemia did not develop during the entire course of pregnancy.

The concentration of tacrolimus in the cord blood was lower than or equal to the detection limit value according to chemiluminescence immunoassay (ECLIA), and the anti-D antibody titer was double. The blood type of the child was type A Rho (D) positive, the Hb (hemoglobin) level at the time of birth was 13.6, which was a slightly low value, and there were no external malformations or visceral malformations and no problem with physical functions.

### Analysis

Fetal hemoglobin (HbF) can exist in maternal blood from the early stage of pregnancy. HbF in maternal blood is detected in the early stages of pregnancy, and the migration of HbF in fetal blood to maternal blood is generally observed from about 9 weeks of pregnancy [Reference Literatures 4, 11, and 12]. These findings suggest that the maternal immune response to fetal antigens can occur from the early stage of pregnancy, in which inflow of the fetal antigens into the mother's body begins. On the other hand, the migration of anti-D antibody to the fetus begins after completion of the placental construction.

Since this patient did not have anti-D immunoglobulin having neutralizing activity, administered to the mother's body during the first pregnancy, there was a possibility that a large amount of fetal blood flowed into the mother's body during delivery due to placental abruption, and it is considered that sensitization to the fetal antigens was strongly established. In addition, there is a high possibility that a general sterility treatment did not result in pregnancy and recurrent failures further promoted sensitization. A possibility was considered in which due to these happenings, fetal antigens including blood cell components were strongly recognized by the mother's body, and rejection caused by not only humoral immunity represented by anti-D antibody but also cell-mediated immune response to other fetal components due to a marked increase in Th1, began from the second pregnancy activity and caused infecundity.

The elevated anti-D antibody titer and the increased Th1 cell population were induced by humoral immunity and cell-mediated immunity, respectively. These are the conventional immune responses to exogenous antigens and fetal antigens.

The inventors could treat patients using tacrolimus and suppress cell-mediated immunity against infecundity. Detailed mechanisms and results related to Th1 cells have been previously described [Reference Literatures 13 to 15]. Based on these results, administration of tacrolimus was initiated before pregnancy as a treatment for infecundity, administration was continued because a decreasing tendency was not observed in the Th1 cell population even after the establishment of pregnancy, and the dose was increased to 5 mg/day because the Th1 cell population increased in week 28 of pregnancy. The maternal and fetal conditions were stable without complications, the pregnancy progressed favorably, and the patient achieved a safe delivery.

After week 24 of pregnancy, there was a possibility that the anti-D antibody titer could rise sharply in response to the invasion of a large amount of fetal red blood cells; however, consequently, the production of anti-D antibody was also suppressed, and intensive care for the mother and the child was unnecessary until delivery.

Although this effect was unexpected, the mechanism can be described such that recognition of antigens and production of anti-pathogen antibodies are inhibited by down-regulation of the T cell function by inhibiting the calcineurin-NFAT pathway, and subsequent inhibition of B cell activation and inhibition of antibody production. This can also be thought of as continuous treatment using high doses of intravenous immunoglobulin having an immunosuppressive effect [Reference Literatures 16 to 19].

In conclusion, this treatment provides a benefit of alleviating the promotion of antibody production without conducting intensive treatments such as plasmapheresis, large-quantity γ-globulin therapy, and high-dose steroid therapy. The inventors believe that treatment using tacrolimus is beneficial for allogeneic immune pregnancies.

It is thought that pregnancy was easily established by immunosuppression by administration of tacrolimus, production of anti-D antibody was suppressed, together with stable continuation of pregnancy, by increasing the amount of tacrolimus during pregnancy for further enhancement of rejection immunity, and it was possible to avoid the onset of fetal anemia. It was speculated that this was due to an effect brought by suppression of both cell-mediated immunity and humoral immunity by tacrolimus.

### Example 2

### Treatment of neonatal hemochromatosis

The main purpose of the present therapeutic method was to suppress the recognition of fetal antigens in the mother's body and to lower the ability to produce pathogenic antibodies.

Although the diseases occurring in the fetus are different, as in the case of blood type incompatible pregnancy, fetal antigens flow in from the fetus into the mother's body after the completion of placenta construction and are recognized in the mother's body, pathogenic antibodies against those antigens are produced, and IgG among them migrates to the fetus via the placenta and thereby causes diseases in the fetus. Regarding the present disease, this pathogenic antibody inhibits proteins related to fetal iron metabolism, iron is deposited in the liver, resulting in liver failure, and in many cases, the disease may be severe to the extent that leads to intrauterine fetal death or postnatal death or requires liver transplantation.

The mechanism of this treatment can be described such that recognition of fetal antigens and production of anti-pathogen antibodies are inhibited by suppression of T cell functions caused by inhibition of the calcineurin/NFAT pathway, as well as consequent inhibition of B cell activation and suppression of antibody production.

A fetal antigen that is considered responsible for this disease has not been identified. Considering that a causative antigen flows in from the fetus into the mother's body after completion of the placenta construction, it is assumed to initiate treatment from around week 12 of pregnancy; however, since there are individual differences in the placental construction, and the timing for the migration of fetal antigens is not clear, it is preferable to initiate treatment from the early stage of pregnancy in order to conduct more effective treatment.

In the case of large-quantity γ-globulin therapy for the mother's body, which is the only existing preventive method, it is necessary to initiate the therapy from week 18 and continue administration until delivery, and the medical cost (600,000 yen/week) is extremely high; however, the present treatment method allows treatment to be performed during the entire course of pregnancy at a medical cost of 1/10 or less of the aforementioned cost, while it is also easy to change the amount of administration as appropriate, so that there is a possibility that the medical cost can be further saved.

In the large-quantity γ-globulin therapy for the mother's body, which is the only existing preventative method, since purification is performed from a large amount of collected and pooled blood, there is a risk of being exposed to infections that is highly possibly included in the blood, and particularly parvovirus infection that causes fetal anemia is considered as a problem; however, there is no risk of infection including other viruses in the present treatment method.

In fetal hemochromatosis, during pregnancy, any of miscarriage, premature birth, intrauterine growth restriction, oligohydramnios, fetal movement insufficiency, or placental edema is frequently recognized, and after birth, defective systemic condition from immediately after birth (respiratory and circulatory failure, and the like), retarded fetal growth, hydrops fetalis, signs of liver failure, and the like are recognized. In neonatal hematological findings, coagulopathy, cholestasis, abnormal transaminase levels, and the like are observed. Disseminated intravascular coagulation syndrome not caused by sepsis, high ferritin level high α-fetoprotein level, high transferrin saturation rate are shown, and in image inspection findings, low signals suggesting iron deposition in organs other than the liver are recognized by MRI T2-weighted imaging. Through this treatment, evaluation is enabled by improving these findings for the fetus during pregnancy and the infant after birth.

### Industrial Applicability

According to the present invention, a disease associated with humoral immunity in the materno-fetal relationship, for example, sterility and infertility caused by humoral immunity in the materno-fetal relationship, blood type incompatible pregnancy, or fetal hemochromatosis can be treated or improved, and continuation of pregnancy and delivery of healthy children are enabled.

### (Citation List)

1: American College of Obstetricians and Gynecologists. ACOG Practice Bulletin No.75: Management of Alloimmunization. Obstet Gynecol 2006;108: 457-464.
2: Moise KJ Jr. Management of rhesus alloimmunization in pregnancy. Obstet Gynecol 2002;100:600-611.
3: Wylie BJ, D'Alton ME. Fetomaternal hemorrhage. Obstet Gynecol 2010;115:1039-1051.
4: Pollack W, Ascari WQ, Kochesky RJ, O'Connor RR, Ho TY, Tripodi D. Studies on Rh prophylaxis. 1. Relationship between doses of anti-Rh and size of antigenic stimulus. Transfusion 1971;11:333-339.
5: Mari G, Deter RL, Carpenter RL, Rahman F, Zimmerman R, Moise KJ Jr, Dorman KF, Ludomirsky A, Gonzalez R, Gomez R, Oz U, Detti L, Copel JA, Bahado-Singh R, Berry S, Martinez-Poyer J, Blackwell SC. Noninvasive diagnosis by Doppler ultrasonography of fetal anemia due to maternal red-cell alloimmunization. Collaborative Group for Doppler Assessment of the Blood Velocity in Anemic Fetuses. N Engl J Med 2000;342:9-14.
6: Kwak-Kim J, Chung-Bang HS, Ng SC, Ntrivalas EI, Mangubat CP, Beaman KD, Beer AE, Gilman-Sachs A:Hum Reprod 2003; 18: 767-773.
7: Schjenken JE, Tolosa JM, Paul JW, Clifton VL, Smith R: Croatia, INTECH, 2012, pp211-242.
8: aito S, Nakashima A, Shima T, Ito M: Am J Reprod Immunol 2010; 63: 601-610.
9: Ng SC, Gilman-Sachs A, Thakar P, Beaman KD, Beer AE, Kwak-Kim J: Am J Reprod Immunol 2002; 48: 77-86.
10: Riley JK: Immunol Invest 2008; 37: 395-426.
11: Zilliacus H, Vartiainen E. Ottelin AM. Adult hemoglobin in the blood of very young human embryos. Nature 1962;193:386-387.
12: Clayton EM Jr, Feldhaus WD, Whitacre FE. Fetal erythrocytes in the maternal circulation of pregnant women. Obstet Gynecol 1964;23:915-919.
13: Nakagawa K, Kwak-Kim J, Ota K, Kuroda K, Hisano M, Sugiyama R, Yamaguchi K. Immunosuppression with Tacrolimus Improved Reproductive Outcome of Women with Repeated Implantation Failure and Elevated Peripheral Blood Th1/Th2 Cell Ratios. Am J Reprod Immunol. 2015; 73: 353-61.
14: Nakagawa K, Kwak-Kim J, Kuroda K, Sugiyama R, Yamaguchi K. Immunosuppressive treatment using tacrolimus promotes pregnancy outcome in infertile women with repeated implantation failures. Am J Reprod Immunol. Sep;78(3). doi: 10.1111/aji.12682. Epub 2017 May 3.
15: Nakagawa K, Kuroda K, Sugiyama R, Yamaguchi K. After 12 consecutive miscarriages, a patient received immunosuppressive treatment and delivered an intact baby. Reprod Med Biol. 2017;16:297-301.
16: De la Camara C, Arrieta R, Gonzalez A, Iglesias E, Omenaca F. High-dose intravenous immunoglobulin as the sole prenatal treatment for severe Rh immunization. N Engl J Med 1988;318:519-520.
17: Margulies M, Voto LS, Mathet E, Margulies M. High-dose intravenous IgG for the Treatment of severe rhesus alloimmunization. Vox Sang 1991;61:181-189.
18: Ruma MS, Moise KJ Jr., Kim E, Murtha AP, Prutsman WJ, Hassan SS, Lubarsky SL. Combined plasmapheresis and intravenous immunoglobulin for the treatment of severe maternal red cell alloimmunization. Am J Obstet Gynecol 2007;196: 138e1-138e6.
19: Isojima S, Hisano M, Suzuki T, Sago H, Murashima A and Yamaguchi K. Early plasmapheresis followed by high-dose -globulin treatment saved a severely Rho-incompatible pregnancy. Journal of Clinical Apheresis 2011; 26: 216-8.

## Claims

1. A medicine for treating a disease associated with humoral immunity in the materno-fetal relationship, the medicine comprising, as an active ingredient, a compound selected from the group consisting of:
(i) a compound represented by Formula (I) or a pharmaceutically acceptable salt thereof, 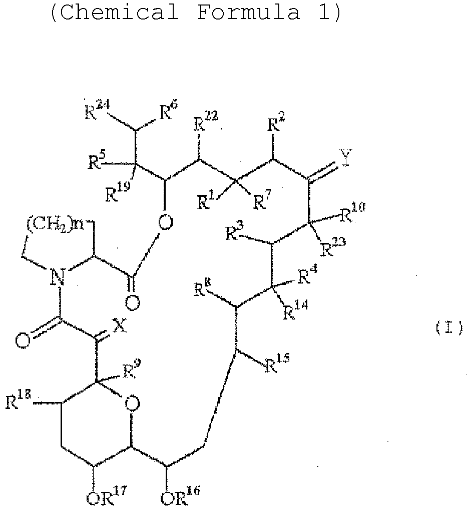 wherein each of the adjacent pairs of R¹ with R², R³ with R⁴, and R⁵ with R⁶ each independently
(a) represents two adjacent hydrogen atoms, or R² may be an alkyl group, or
(b) may form another bond between the carbon atoms to which the pair members are respectively bonded;
R⁷ represents a hydrogen atom, a hydroxy group, or a protected hydroxy group, or may be bonded to R¹ and together represent an oxo group;
R⁸ and R⁹ independently represent a hydrogen atom or a hydroxy group;
R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
X represents an oxo group, (a hydrogen atom, a hydroxy group), (a hydrogen atom, a hydrogen atom), or a group represented by formula: -CH₂O-;
Y represents an oxo group, (a hydrogen atom, a hydroxy group), (a hydrogen atom, a hydrogen atom), or a group represented by formula: N-NR¹¹R¹² or N-OR¹³;
R¹¹ and R¹² independently represent a hydrogen atom, an alkyl group, an aryl group, or a tosyl group;
R¹³, R¹⁴, R¹⁵, R¹⁶, R¹⁷, R¹⁸, R¹⁹, R²², and R²³ independently represent a hydrogen atom or an alkyl group;
R²⁴ represents a ring which can include one or more heteroatoms and may be substituted as desired;
n represents 1 or 2; and
in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof.

2. The medicine according to claim 1, wherein the compound represented by Formula (I) is tacrolimus or a pharmaceutically acceptable salt thereof.

3. The medicine according to claim 1, wherein the active ingredient is a compound represented by Formula (I) or a pharmaceutically acceptable salt thereof, and the compound represented by Formula (I) is tacrolimus or a pharmaceutically acceptable salt thereof.

4. The medicine according to any one of claims 1 to 3, wherein the disease associated with humoral immunity in the materno-fetal relationship is sterility and infertility caused by humoral immunity in the materno-fetal relationship.

5. The medicine according to any one of claims 1 to 3, wherein the disease associated with humoral immunity in the materno-fetal relationship is blood type incompatible pregnancy.

6. The medicine according to any one of claims 1 to 3, wherein the disease associated with humoral immunity in the materno-fetal relationship is fetal hemochromatosis.

7. The medicine according to any one of claims 4 to 6, wherein the medicine is applied to pregnancies of the second and succeeding children.

8. The medicine according to any one of claims 4 to 7, wherein the medicine is administered from early stage of pregnancy.

9. The medicine according to any one of claims 4 to 7, wherein the medicine is administered at a dose of 1 to 10 mg/day from the early stage of pregnancy.

10. The medicine according to claim 9, wherein the medicine is administered at a dose of 3 to 6 mg/day from the early stage of pregnancy.

11. The medicine according to any one of claims 1 to 3, 5, and 7 to 9, wherein the medicine is administered to a patient having a potential for blood type incompatible pregnancy in an amount of administration of 1 to 10 mg/day from the early stage of pregnancy.
